# NEUE EUROPÄISCHE PATENTSCHRIFT

(11) **EP 2 138 154 B2**
(45) Veröffentlichungstag und Bekanntmachung des Hinweises auf die Entscheidung über den Einspruch: **07.08.2024**
(45) Hinweis auf die Patenterteilung: 25.07.2018
(21) Anmeldenummer: 09160970.1
(22) Anmeldetag: 25.05.2009
(51) Int. Cl.: A61K 8/42, A61K 8/44, A61K 8/63, A61K 8/73, A61K 8/891, A61Q 5/12

(54) **Haarspülung mit Amphotensid und besonderer Lagerstabilität**
Hair rinse with amphotenside and special storage life
Après-shampoing doté d'amphotenside et d'une stabilité de stockage particulière

(30) Priorität: 27.06.2008 DE 102008030138
(43) Veröffentlichungstag der Anmeldung: 30.12.2009
(73) Patentinhaber: Beiersdorf AG, 22529 Hamburg (DE)
(72) Erfinder: Demitz, Michael, 22529 Hamburg (DE); Köhler, Manuela, 22147 Hamburg (DE); Saladin, Sandra, 20144 Hamburg (DE); Mahadeshwar, Anand, 22529 Hamburg (DE); Wendicke, Silke, 22303 Hamburg (DE)
(74) Vertreter: Beiersdorf AG

(56) Entgegenhaltungen:
- EP-A1- 0 562 637
- EP-A1- 2 116 225
- EP-A1- 2 116 226
- WO-A1-94/04125
- DE-A1- 10 315 715
- DE-A1- 19 754 283
- DE-A1- 3 024 578
- JP-A- H05 271 036
- JP-A- H05 271 036
- US-A- 4 986 983
- US-A1- 2006 024 256
- US-A1- 2006 293 213
- Maschinenübersetzung von JPH05271036 XP055353997

## Beschreibung

Haarspülungen werden zur Pflege nach der Reinigung der Haare angewendet. Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar.

"Sensorik" im Sinne dieser Schrift umfasst alle fühlbaren und für den Anwender relevanten Eigenschaften des Produktes bei Entnahme des Produktes aus der Packung, bestimmungsgemäßer Anwendung (Einmassieren in das Haar, Aufenthalt des Produktes im Haar, Ausspülen) sowie die während und nach der Anwendung fühlbaren Veränderungen an Haar, Kopfhaut und Fingern. Insbesondere umfasst "Sensorik" die Eigenschaften "Gehaltvoll", "Gleitvermögen", "Entwirrbarkeit", "Kämmbarkeit", "Haarstruktur", "Weich geschmeidig".

"Gehaltvoll" im Sinne dieser Schrift wird wie folgt festgelegt: Es wird gemessen in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibenden Druck an der Haarsträhne von oben nach unten. Hierbei wird eine 10er Skala verwendet bei der 10 das höchstmögliche Gleitvermögen darstellt.

"Entwirrbarkeit" im Sinne dieser Schrift ist die Messung wie leicht sich die Haare nach dem Ausspülen des Produkts mit dem Kamm entwirren lassen. Die Haare sind dann entwirrt, wenn der Kamm einmal komplett von oben nach unten durch die Haarsträhne geführt werden kann. Hierbei wird eine 10er Skala verwendet bei der 10 die höchstmögliche Entwirrbarkeit darstellt.

"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

"Haarstruktur" im Sinne dieser Schrift bedeutet die Beurteilung des Haares durch horizontales Rollen der Haare zwischen Daumen und Zeigefinger/ Mittelfinger einer Hand. Dies wird an verschiedenen Stellen der Strähne beginnend von oben nach unten geprüft. Hierbei wird eine 10er Skala verwendet bei der 10 die höchstmögliche Haarstruktur darstellt.

"Weich geschmeidig" im Sinne dieser Schrift bedeutet das sensorische Empfinden nach Applikation des Produktes im nassen und trockenen Haar durch einen Experten Panel.

Silikonemulsionen sind Zubereitungen, die in Wasser dispergierte oder emulgierte Silikonöle enthalten. Solche Silikonöle sind beispielsweise Polidimethylsiloxane. Diese sind oft durch oberflächenaktive Substanzen dispergiert oder emulgiert. Geeignete Emulgatoren sind Cocoamidopropylbettaoin, C12-C15 Pareth-3 oder Guarhydroxytrimoniumchlorid. Die Tröpchengrößen bewegen sich im Bereich einiger Mikrometer, insbesondere im Bereich von 15 - 30 Mikrometern.

Kationische Polymere sind Makromolekulare Substanzen, welche in der Haupt- und/oder Seitenkette eine Gruppe aufweisen, welche "temporär" oder "permanent" kationisch sein kann.

Geeignete kationische Polymere sind beispielsweise quaternisierte Cellulose-Derivate, wie sie unter den Bezeichnungen Celquat und Polymer JR im Handel erhältlich sind. Die Verbindungen CelquatH 100, CelquatL 200 und Polymer JR400 sind bevorzugte quaternierte Cellulose-Derivate, kationische Alkylpolyglycoside gemäss der DE 44 13 686, kationisierter Honig, beispielsweise das Handelsprodukt Honeyquat 50, kationische Guar-Derivate, wie insbesondere die unter den Handelsnamen Cosmedia Guar und Jaguarvertriebenen Produkte, polymere Dimethyldiallylammoniumsalze und deren Copolymere mit Estern und Amiden von Acrylsäure und Methacrylsäure. Die unter den Bezeichnungen Merquat 100 (Poly(dimethyldiallylammoniumchlorid)) und Merquat550 (Dimethyldiallylammoniumchlorid-Acrylamid-Copolymer) im Handel erhältlichen Produkte sind Beispiele für solche kationischen Polymere, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat 734 und Gafquat 755 im Handel erhältlich, Vinylpyrrolidon-Vinylimidazoliummethochlorid-Copolymere, wie sie unter den Bezeichnungen Luviquat FC 370, FC 550, FC 905 und HM 552 angeboten werden, quaternierter Polyvinylalkohol, sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette, die unter den Bezeichnungen Polyquaternium-24 (Handelsprodukt z. B. Quatrisoft LM 200), bekannten Polymere, die Copolymere des Vinylpyrrolidons, wie sie als Handelsprodukte Copolymer 845 (Hersteller: ISP), Gaffix VC 713 (Hersteller: ISP), Gafquat ASCP 1011, GafquatHS 110, Luviquat8155 und Luviquat MS 370 erhältlich sind.

US 4507280 offenbart Alkyl-Betaine mit 10 bis 24 Kohlenstoffatomen in haarkonditionierenden Zubereitungen WO 2006136303 offenbart Konditioniermittel für keratinhaltige Fasern enthaltend u. a. amphoteres Tensid (Disodium Cocoamphodiacetat). US 4986983 offenbart haarkonditionierende Zubereitungen enthaltend u. a. Betaine. Die frühere Europäische Patentanmeldung EP2116226 A1 offenbart Haarspülungen enthaltend Stearamidopropyl Dimethylamine und Coco Betaine.

Die japanische Patentanmeldung JPH05271036 offenbart viskositätsstabile Haarbehandlungsmittel u.a. enthaltend Amidoamine und ampholytische Tenside. Eine Haarspülung soll aus Konsumentensicht reichhaltig und cremig sein, das Haar pflegen, das Haar im nassen und trockenen Zustand weich, geschmeidig, gut kämmbar, glänzend machen und die Spülung muss schnell wirken.

Haarspülungen sind normalerweise angedickte Gele oder Cremes, die eine leichte Verteilbarkeit und Anwendbarkeit auf dem Haar gewährleisten. Es ist besonders wichtig, dass die Viskosität des Produktes über die Zeit konstant bleibt. Ein Anstieg der Viskosität der Formel hat einen negativen Effekt auf die Produktentnahme aus der Flasche. Außerdem ist es wichtig die Viskosität des Produktes im Tonnenmaßstab zu kontrollieren. Es wurde festgestellt, dass die Viskosität von Haarspülungen, die Amidoamine enthalten über 180 Tage deutlich ansteigt.

Daher hat sich überraschend gezeigt, dass eine kosmetische Haarbehandlungszubereitung vorgesehen für das Ausspülen nach der Anwendung mit Amidoaminen und 0,0001 bis 1 % Aktivgehalt an amphoteren Tensiden und Guar Hydroxypropyltrimonium Chlorid, wobei als amphoteres Tensid Coco Betaine enthalten ist und als Amidoamin Stearamidopropyl Dimethylamine eingesetzt wird, den Nachteilen des Standes der Technik abhilft.

Eine Zugabe von einer kleinen Menge (<1%) von Coco-Betain hat einen stabilisierenden Effekt auf die Stabilität einer Spülungsformel, die Amidoamine enthält, was bedeutet, dass die Viskosität konstant bleibt. Des Weiteren wurde ein sensorischer Vorteil im trockenen und nassen Zustand des Haares beobachtet, der sich in der Produktreichhaltigkeit, verbesserten Verteilbarkeit, sowie Trocken-kämmbarkeit und Geschmeidigkeit und der allgemeinen Haarstruktur im Halbseiten-Haarstudiotest auf trockenem/strapaziertem Haar an 10 Personen zeigt. Dabei ist es von Vorteil, wenn zusätzlich 0,1 bis 1 Gew.-% Oryzanol enthalten sind. Dabei ist es weiter von Vorteil, wenn

Amidoamin in Konzentrationen von 1.5 bis 2.5 Gew.-% eingesetzt wird. Dabei ist es weiter von Vorteil, wenn als Amidoamin Stearamidopropyl Dimethylamine eingesetzt wird. Dabei ist es weiter von Vorteil, wenn kationische Polymere in Gehalten von 0,4 bis 0,7 Gew.-% vorhanden in einer hochmolekularen Silikonemulsion mit Partikelgrößen von 10 bis 50 Mikrometern enthalten sind. Es ist als kationisches Polymer Guar Hydroxypropyltrimonium Chlorid vorhanden, bevorzugt mit einer mittleren Ladungsdichte von 0.2 bis 1 meq per gram. Dabei ist es weiter von Vorteil, wenn die Silikon-emulsion eine Viskosität 6000 bis 12000 mPas (Brookfield, Spindel 6, 50 RPM). Dabei ist es weiter von Vorteil, wenn die Zubereitung frei von Cellulosederivaten ist. Dabei ist es weiter von Vorteil, wenn die Zubereitung nicht waschaktiv ist. Dabei ist es weiter von Vorteil, wenn die Zubereitung frei von anionischen Tensiden ist. Dabei ist es weiter von Vorteil, wenn die Zubereitung außer kationischen keine weiteren Polymere enthält.

Erfindungsgemäße Zubereitungen können zusätzlich UV- Filter, Polyole, Konservierungsmittel, hydrolysierte Proteine, Pflanzenextrakte, Parfümöle, und Antioxidantien sowie auch amphotere Tenside enthalten.

Besonders bevorzugte erfindungsgemäße Mittel Fasern sind dadurch gekennzeichnet, dass sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel Coenzym Q10 enthalten

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons, besonders bevorzugt Mittel Plastochinon PQ-9 enthalten.

Es ist bekannt dass besonders tertiäre Amine in Kombination mit den Fettalkoholen Cetyl und Stearyl Alkoholen zu reichhaltigen, cremigen Spülungsgrundlagen führen, wie in EP 0859587B1, EP617953B1, WO2002102334A2 und EP1586298B1 beschrieben.

Aus Tabelle 1 sieht man, dass die Viskosität von Vergleichsformel 1 um 4500mPas in 180 Tagen ansteigt. Die Viskosität von Formel 2, die 0.85%wt Coco-Betain enthält, steigt um 1300mPas und Vergleichsformel 3 mit 0.74%wt Cocamidopropylbetain steigt um 2500mPas an. Somit ist Coco-Betain effektiver, um eine konstante Viskosität von der Spülungsformel enthaltend Amidoamine zu gewährleisten. Diese Formel wurden im Labormaßstab (1-2.5kg) mittels eines Kitchen Aids^{®} hergestellt.

| | **wt %** | **1** | **2** | **3** |
|---|---|---|---|---|
| 1 | Methylparaben | 0,25 | 0,25 | 0,25 |
| 2 | Propylparaben | 0,10 | 0,10 | 0,10 |
| 3 | Aqua | 83,99 | 83,14 | 83,25 |
| 4 | Cetyl Alcohol | 2,50 | 2,50 | 2,50 |
| 5 | Lactic Acid | 0,80 | 0,80 | 0,80 |
| 6 | Sodium Chloride | 0,01 | 0,01 | 0,01 |
| 7 | Stearyl Alcohol | 4,80 | 4,80 | 4,80 |

| | **wt %** | **1** | **2** | **3** |
|---|---|---|---|---|
| 8 | Cocamidopropylbetaine | | | 0,74 |
| 9 | Oryzanol | 0,05 | 0,05 | 0,05 |
| 10 | Coco Betaine | | 0,85 | |
| 11 | Stearamidopropyl Dimethylamine | 2,20 | 2,20 | 2,20 |
| 12 | Parfum | 0,70 | 0,70 | 0,70 |
| 13 | Dimethicone + Cocamidopropyl Betaine + Guar Hydroxypropyl-trimonium Chloride + C12-15 Pareth-3 | 4,60 | 4,60 | 4,60 |
| t₃₀ values (mPas) | pH (90d) | 4,06 | 3,88 | 3,90 |
| | viscosity (1d) | 2800 | 2300 | 1700 |
| | viscosity (3d) | 3050 | 2550 | 4200 |
| | viscosity (14d) | 6300 | 2900 | 4100 |
| | viscosity (28d) | 6450 | 3150 | 4100 |
| | viscosity (90d) | 6900 | 3250 | 3700 |
| | viscosity (180d) | 7300 | 3600 | 4200 |

| **Tabelle 1** | | | |
|---|---|---|---|
| Viscosity measured with HAAKE viscotester VT02 with Rotor 1 | | | |

### Handelsnamen der Rohstoffe

| | | | | |
|---|---|---|---|---|
| 1 | Nipagin M, Clariant, 99.5% | | | |
| 2 | Nipasol M, Clariant, 99.5% | | | |
| 4 | Nacol 16-95, Sasol, 95% | | | |
| 5 | 100366 Milchsäure 90, reinst DAB, Merck, 90% | | | |
| 7 | Lanette 18, Cognis, 100% | | | |
| 8 | Tego-Betain F50, Evonik Goldschmidt, 34% | | | |
| 9 | Gamma-Oryzanol, Biesterfeld, 98% | | | |
| 10 | Dehyton AB 30, Cognis, 31% | | | |
| 11 | Tego Amid S18, Evonik Goldschmidt, 100% | | | |
| 13 | DC 5-7139, 65% Dimethicone | | | |

In Tabelle 2 sind Spülungen, die im Technikumsmaßstab (12kg) mittels einer Prozessanlage Becomix 15CD hergestellt worden sind, abgebildet. Bei der Herstellung im Becomix wird die Lipidphase zur Wasserphase gegeben und die Homogenisierung erfolgt mit einer Umpumpleitung (Rotor-Stator-System mit 1 Rotor- und 1 Statorkränzen). Ohne Coco-Betain steigt die Viskosität von der Vergleichsspülungsformel 4 um 3300mPas innerhalb 180 Tage an, wogegen die Viskosität von Formel 5 nur um 550mPas ansteigt. Die Kontrolle der Viskosität für Produktion im technischen Maßstab ist essentiell, um die Produktqualität, gleich bleibende sensorische Eigenschaften und Performance zu gewährleisten.

| | **wt %** | **4** | **5** |
|---|---|---|---|
| 1 | Methylparaben | 0,25 | 0,25 |
| 2 | Propylparaben | 0,10 | 0,10 |
| 3 | Aqua | 83,99 | 83,14 |
| 4 | Cetyl Alcohol | 2,50 | 2,50 |
| 5 | Lactic Acid | 0,80 | 0,80 |
| 6 | Sodium Chloride | 0,01 | 0,01 |
| 7 | Stearyl Alcohol | 4,80 | 4,80 |
| 8 | Oryzanol | 0,05 | 0,05 |
| 9 | Coco Betaine | | 0,85 |
| 10 | Stearamidopropyl Dimethylamine | 2,20 | 2,20 |
| 11 | Parfum | 0,70 | 0,70 |
| 12 | Dimethicone + Cocamidopropyl Betaine + Guar Hydroxypropyl-trimonium Chloride + C12-15 Pareth-3 | 4,60 | 4,60 |
| | pH (90d) | 4,05 | 4,10 |
| t₃₀ values (mPas) | viscosity (1d) | 4000 | 3500 |
| | viscosity (3d) | 5000 | 4000 |
| | viscosity (14d) | 6000 | 4000 |
| | viscosity (28d) | 6000 | 4100 |
| | viscosity (90d) | 6500 | 4500 |
| | viscosity (180d) | 7300 | 4050 |

| **Tabelle 2** | | | |
|---|---|---|---|
| **Handelsnamen der Rohstoffe** | | | |
| 1 | Nipagin M, Clariant, 99.5% | | |
| 2 | Nipasol M, Clariant, 99.5% | | |
| 4 | Nacol 16-95, Sasol, 95% | | |
| 5 | 100366 Milchsäure 90, reinst DAB, Merck, 90% | | |
| 7 | Lanette 18, Cognis, 100% | | |
| 8 | Gamma-Oryzanol, Biesterfeld, 98% | | |
| 9 | Dehyton AB 30, Cognis, 31% | | |
| 10 | Tego Amid S18, Evonik Goldschmidt, 100% | | |
| 12 | DC 5-7139, 65% Dimethicone | | |

Die Zugabe von Coco-Betain in Spülungen führt zu einer verbesserten Konditionierleistung, wie in Tabelle 3 abgebildet, für Formel 2, besonders in Produktverteilbarkeit, Reichhaltigkeit beim Verteilen im nassen Haar sowie auch im trockenen Zustand des Haares.

### Haarstudio Daten; Halbseiten-Test an trocken/strapazierten Haaren, N=10 Probanden

| **Tabelle 3** | | |
|---|---|---|
| | **1** | **2** |
| Verteilbarkeit (nass) | 7,30 | 7,50 |
| Gehalt verteilen | 7,40 | 7,60 |
| Frisurenfülle | 7,30 | 7,40 |
| Entwirrbarkeit trocken | 6,80 | 7,20 |
| Kämmbarkeit trocken | 7,10 | 7,50 |
| Gleitfähigkeit trocken | 7,00 | 7,10 |
| Struktur trocken Längen | 7,30 | 7,50 |
| Struktur trocken Spitzen | 6,40 | 6,70 |
| weich geschmeidig trocken | 1,00 | 2,00 |

### Weitere Formelbeispiele:

Die Beispiele 6, 7, 9 und 10 sind Vegleichsbeispiele.

| | Wt% | **6** | **7** | **8** | **9** | **10** |
|---|---|---|---|---|---|---|
| 1 | Methylparaben | 0,25 | 0,25 | 0,25 | 0,25 | 0,25 |
| 2 | Propylparaben | 0,10 | 0,10 | 0,10 | 10 | 0,10 |
| 3 | Aqua | 84,46 | 86,37 | 85,22 | 85,27 | 81,56 |
| 4 | Cetyl Alcohol | 2,40 | 1,88 | 1,88 | 2,25 | 3,00 |
| 5 | Dimethicone | | 3,00 | | 3,00 | 3,00 |
| 6 | Panthenol | 0,15 | | | | |
| 7 | Lactic Acid | 0,83 | 0,70 | 0,70 | 0,70 | 0,70 |
| 8 | Sodium Chloride | 0,01 | 0,01 | 0,01 | 0,01 | 0,01 |
| 9 | Stearyl Alcohol | 4,80 | 3,60 | 3,60 | 4,32 | 5,76 |
| 10 | Oryzanol | 0,05 | 0,05 | 0,05 | 0,05 | 0,05 |
| 11 | Cyclomethicone | 2,70 | | | | |
| 12 | Coco Betaine | 0,85 | 1,70 | 0,85 | 1,70 | 3,22 |
| 13 | Stearamidopropyl Dimethylamine | 2,20 | 1,65 | 1,65 | 1,65 | 1,65 |
| 14 | Parfum | 0,70 | | | | |
| 15 | Parfum | | 0,70 | 0,70 | 0,70 | 0,70 |
| 16 | Dimethicone | 0,50 | | | | |
| 17 | Dimethicone + Cocamidopropyl Betaine + C12-15 Pareth-3 + Guar Hydroxypropyltrimonium Chloride | | | 5,00 | | |

### Handelsnamen der Rohstoffe

| | |
|---|---|
| 1 | Nipagin M, Clariant, 99.5% |
| 2 | Nipasol M, Clariant, 99.5% |
| 4 | Nacol 16-95, Sasol , 95% |
| 5 | DC 200 Fluid 100cst, Dow Corning, 100% |
| 6 | Dexpanthenol 75% aq. Solution, Daiichi Fine Chemical, 77%ig |
| 7 | 100366 Milchsäure 90, reinst DAB, Merck, 90% |
| 9 | Lanette 18, Cognis , 100% |
| 10 | Gamma -Oryzanol, Biesterfeld, 98% |
| 11 | Dow Corning 245 Fluid, Dow Corning, 100% |
| 12 | Dehyton AB 30, Cognis, 31% |
| 13 | Tego Amid S18, Evonik Goldschmidt, 100% |
| 16 | DC 200 Fluid 12500cst, Dow Corning, 100% |
| 17 | DC 5-7139, Dow Corning, 65% Dimethicone |

## Patentansprüche

1. Kosmetische Haarbehandlungszubereitung vorgesehen für das Ausspülen nach der Anwendung mit Amidoaminen, 0,0001 bis 1% Aktivgehalt an amphoteren Tensiden und Guar Hydroxypropyltrimonium Chlorid, wobei als amphoteres Tensid Coco Betaine enthalten ist und als Amidoamin Stearamidopropyl Dimethylamine eingesetzt wird.

2. Zubereitung nach Anspruch 1 **dadurch gekennzeichnet, dass** zusätzlich 0,1 bis 1 Gew.% Oryzanol enthalten sind.

3. Zubereitung nach einem der vorangehenden Patentansprüche **dadurch gekennzeichnet, dass** Amidoamin In Konzentrationen von 1,5 bis 2,5 Gew.% eingesetzt wird.

4. Zubereitung nach einem der vorangehenden Patentansprüche **dadurch gekennzeichnet, dass** kationische Polymere in Gehalten von 0,4 bis 0,7 Gew.% vorhanden in einer hochmolekularen Silikonemulsion mit Partikelgrößen von 10 bis 50 Mikrometern enthalten sind.

5. Zubereitung nach einem der vorangehenden Patentansprüche **dadurch gekennzeichnet, dass** als kationisches Polymer Guar Hydroxypropyltrimonium Chlorid mit einer mittleren Ladungsdichte von 0.2 bis 1 meq per gram verwendet wird.

6. Zubereitung nach einem der vorangehenden Patentansprüche **dadurch gekennzeichnet, dass** die Silikonemulsion eine Viskosität 6000 -12000 mPas (Brookfield, Spindel 6, 50 RPM).

7. Zubereitung nach einem der vorangehenden Patentansprüche **dadurch gekennzeichnet, dass** die Zubereitung frei von Cellulosederivaten ist.

8. Zubereitung nach einem der vorangehenden Patentansprüche **dadurch gekennzeichnet, dass** die Zubereitung nicht waschaktiv ist.

9. Zubereitung nach einem der vorangehenden Patentansprüche **dadurch gekennzeichnet, dass** die Zubereitung frei von anionischen Tensiden ist,

10. Zubereitung nach einem der vorangehenden Patentansprüche **dadurch gekennzeichnet, dass** die Zubereitung außer kationischen keine weiteren Polymere enthält,

## Claims

1. Cosmetic hair treatment preparation, which is intended to be rinsed off after application, with amidoamines, 0.0001 to 1% active content of amphoteric surfactants and guar hydroxypropyltrimonium chloride, wherein coco betaine is present as amphoteric surfactant and stearamidopropyl dimethylamine is used as amidoamine.

2. Preparation according to Claim 1, **characterized in that** in addition 0.1 to 1 wt% oryzanol is present.

3. Preparation according to either of the preceding patent claims, **characterized in that** amidoamine is used at concentrations of 1.5 to 2.5 wt%.

4. Preparation according to any of the preceding patent claims, **characterized in that** cationic polymers are present at contents of 0.4 to 0.7 wt% in a high molecular weight silicone emulsion having particle sizes of 10 to 50 micrometres.

5. Preparation according to any of the preceding patent claims, **characterized in that** the cationic polymer used is guar hydroxypropyltrimonium chloride with an average charge density of 0.2 to 1 meq per gram.

6. Preparation according to any of the preceding patent claims, **characterized in that** the silicone emulsion a viscosity of 6000 - 12 000 mPas (Brookfield, spindle 6, 50 rpm).

7. Preparation according to any of the preceding patent claims, **characterized in that** the preparation is free of cellulose derivatives.

8. Preparation according to any of the preceding patent claims, **characterized in that** the preparation is not washing-active.

9. Preparation according to any of the preceding patent claims, **characterized in that** the preparation is free of anionic surfactants.

10. Preparation according to any of the preceding patent claims, **characterized in that** the preparation comprises no further polymers other than cationic polymers.

## Revendications

1. Préparation cosmétique de traitement capillaire prévue pour le rinçage après l'application, comportant des amidoamines, une teneur en tensioactifs amphotères de 0,0001 à 1 % d'actifs et du Guar Hydroxypropyl-trimonium Chloride, dans laquelle de la Coco Betaine est contenue en tant que tensioactif amphotère et de la Stearamidopropyl Dimethylamine est utilisée en tant qu'amidoamine.

2. Préparation selon la revendication 1, **caractérisée en ce que** 0,1 à 1 % en poids d'oryzanol est en outre contenu.

3. Préparation selon l'une quelconque des revendications précédentes, **caractérisée en ce que** l'amidoamine est utilisée à des concentrations de 1,5 à 2,5 % en poids.

4. Préparation selon l'une quelconque des revendications précédentes, **caractérisée en ce que** des polymères cationiques sont contenus en des teneurs de 0,4 à 0,7 % en poids dans une émulsion de silicone de masse moléculaire élevée à tailles de particules de 10 à 50 micromètres.

5. Préparation selon l'une quelconque des revendications précédentes, **caractérisée en ce que** du Guar Hydroxypropyltrimonium Chloride ayant une densité moyenne de charge de 0,2 à 1 mEq par gramme est utilisé en tant que polymère cationique.

6. Préparation selon l'une quelconque des revendications précédentes, **caractérisée en ce que** l'émulsion de silicone une viscosité de 6 000 - 12 000 mPa.s (Brookfield, broche 6, 50 tours/min).

7. Préparation selon l'une quelconque des revendications précédentes, **caractérisée en ce que** la préparation est exempte de dérivés de cellulose.

8. Préparation selon l'une quelconque des revendications précédentes, **caractérisée en ce que** la préparation n'est pas lavante.

9. Préparation selon l'une quelconque des revendications précédentes, **caractérisée en ce que** la préparation est exempte de tensioactifs anioniques.

10. Préparation selon l'une quelconque des revendications précédentes, **caractérisée en ce qu'**hormis des polymères cationiques la préparation ne contient pas d'autres polymères.
